# EUROPEAN PATENT SPECIFICATION

(11) **EP 4 033 926 B1**
(45) Date of publication and mention of the grant of the patent: **28.06.2023**
(21) Application number: 20775724.6
(22) Date of filing: 17.09.2020
(51) Int. Cl.: A24F 42/20, A24F 42/60, A24F 42/80, A61M 15/06

(54) **INHALER ARTICLE WITH A TWISTED DISTAL END ELEMENT**
INHALATORARTIKEL MIT EINEM VERDRILLTEN ELEMENT MIT DISTALEM ENDE
ARTICLE INHALATEUR DOTÉ D'UN ÉLÉMENT D'EXTRÉMITÉ DISTALE TORSADÉE

(30) Priority: 26.09.2019 EP 19199962
(43) Date of publication of application: 03.08.2022
(73) Proprietor: Philip Morris Products S.A., 2000 Neuchâtel (CH)
(72) Inventor: CAMPITELLI, Gennaro, 2000 Neuchâtel (CH); COUDERC, Gaetan, 2000 Neuchâtel (CH)
(74) Representative: Kelvey, Adam Charles
(86) International application number: PCT/IB2020/058685
(87) International publication number: WO 2021/059094

(56) References cited:
- WO-A1-2012/105236
- WO-A1-2018/100461
- WO-A1-2019/003118
- WO-A1-2019/082057
- WO-A1-2019/159123
- US-A1- 2019 282 769

## Description

This disclosure relates to an inhaler article having a twisted distal end element and to a method of making the twisted distal end element.

Dry powder inhalers are not always fully suitable to provide dry powder particles to the lungs at inhalation or air flow rates that are within conventional smoking regime inhalation or air flow rates. Dry powder inhalers may be complex to operate or may involve moving parts. Dry powder inhalers often strive to provide an entire dry powder dose in a single breath.

Dry powder inhalers are typically formed of materials that are not biodegradable. In addition, these dry powder inhalers are often formed of materials that may need to be injection moulded or cast and assembly of these parts may present a bottleneck in the manufacturing process and are difficult to produce at high speeds.

WO 2019/082057 A1 discloses an inhaler system including an inhaler article and a piercing article. The piercing article contains a recessed piercing element and is configured to receive a distal end of the inhaler article. The piercing element pierces or punctures a single hole into a capsule contained within the inhaler article when the inhaler article is seated into the piercing article.

WO 2019/159123 A1 discloses a dry powder inhaler for providing pharmaceutically active particles including a mouthpiece portion removably coupled to a distal end portion. The mouthpiece portion extends between the mouthpiece end and a first mating end. The distal end portion extends between a second mating end and the distal end. A capsule cavity is defined within the first mating end. An airflow element is disposed within the distal end portion and extends a longitudinal length to the capsule cavity. The airflow element includes helical airflow channels rotating about the circumference of the airflow element and extending the longitudinal length of the airflow element and non-parallel with the longitudinal axis, the airflow channels directing inlet air into the capsule cavity to produce swirling air flow to induce rotation of a capsule contained within the capsule cavity, and a resealable membrane configured to reseal when a piercing element is withdrawn from the resealable membrane.

It would be desirable to provide an inhaler article that may be assembled at high speeds. It would also be desirable to provide an inhaler article that has a form that is easy to hold and is familiar to a user, similar to a conventional cigarette. It would also be desirable to provide an inhaler article that is convenient to use by a consumer. It would be desirable to provide a dry powder inhaler that is substantially biodegradable.

The invention is defined in the independent claims 1 and 13. Preferred embodiments are matter of the dependent claims.

According to an aspect of the present invention, there is provided an inhaler article comprising: a body extending along a longitudinal axis from a mouthpiece end to a distal end of the inhaler article; a capsule cavity defined within the body; and a distal end element disposed at the distal end and extending to the capsule cavity. The distal end element comprises an element distal end, an element inner end, a solid core portion, and at least two helical grooves. The solid core portion extends from the element distal end to the element inner end. The at least two grooves are helical grooves that rotate about the solid core portion along the longitudinal axis from the element distal end to the element inner end. The at least two helical grooves extend along an outer surface of the distal end element.

According to an aspect of the present invention, there is provided an inhaler article comprising: a body extending along a longitudinal center axis from a mouthpiece end to a distal end of the inhaler article; a capsule cavity defined within the body; and a distal end element disposed at the distal end and extending to the capsule cavity. The distal end element comprises an element distal end, an element inner end, a solid core portion, and at least two helical grooves. The solid core portion extends from the element distal end to the element inner end. The at least two grooves are helical grooves that rotate about the solid core portion along the longitudinal center axis from the element distal end to the element inner end. The at least two helical grooves extend along an outer surface of the distal end element.

The term "solid core portion" refers to a core portion of the distal end element that is solid; that is, that the core portion does not have an internal cavity or that the core portion is not hollow or that the core portion does not have any substantial gaps, channels or passages. The solid core portion may be formed of a homogeneous material along the entire length of the distal end element. The solid core portion may be formed of a homogeneous porous material along the entire length of the distal end element. Such core portion comprises a central portion of the distal end element.

A "substantial gap, channel, or passage" is understood to mean an opening that is greater in size than the pores (if any) of the material the end element is formed of. A substantial gap, channel, or passage is understood to mean an opening that has a lateral dimension of 1 mm or greater.

There is also provided an inhaler article comprising: a body extending along a longitudinal axis from a mouthpiece end to a distal end; a capsule cavity defined within the body; and a distal end element disposed at the distal end of the inhaler article and extending to the capsule cavity. The distal end element comprises an element distal end, an element inner end and at least two helical grooves on an outer surface of the distal end element extending from the element distal end to the element inner end. The distal end element having at least two grooves may be twisted to form a twisted distal end element. Preferably, such at least two grooves of the distal end element material are substantially linear prior to twisting and helical following twisting.

Preferably, the distal end element is a distal end plug.

The solid core portion extends from the element distal end to the element inner end and form a continuous solid core. The at least two grooves may be parallel grooves that rotate about continuous core along the longitudinal center axis from the element distal end to the element inner end. The at least two helical grooves extend along an outer surface of the distal end element. The solid core portion may be formed of a porous material. Preferably the solid core portion is formed of cellulose material, such as cellulose acetate. Preferably, the solid core portion is formed of a polylactic acid material.

Preferably the distal end element is formed of a biodegradable material. Preferably the distal end element comprises a fibrous material. Preferably the distal end element is formed of a porous material. Preferably the distal end element is formed of a cellulose material, such as cellulose acetate. Preferably, the distal end element is formed of a polylactic acid material.

Advantageously, distal end element may be formed of materials used to assemble conventional cigarettes. Advantageously, the inhaler article may be formed of biodegradable materials.

The term "biodegradable" refers to a material can be broken down by bacterial decomposition to result in natural byproducts such as gasses (CO₂ and N₂), water, biomass and inorganic salts. Biodegradable materials include materials derived from plants such as polysaccharides, cellulose, cellulose acetate, starch gums, materials derived from microorganisms such as PolyHydroxy Alkanoates (PHA), and synthetic materials such as polylactides and polylactic acids (PLA). These biodegradable materials can be fibrous or porous. The distal end element may be formed of a biodegradable material, a fibrous material or a porous material. The biodegradable material may be, for example, cellulose, cellulose acetate or polylactic acid.

Preferably the parallel grooves may rotate at least 90 degrees from the element distal end to the element inner end. The parallel grooves may rotate at least 180 degrees from the element distal end to the element inner end. Preferably the element distal end is substantially aligned with the distal end of the inhaler article or body.

Advantageously, the distal end element may provide or produce a "swirling" effect on the inhalation airflow into the capsule cavity that may be useful for effective depletion of the capsule during use. Advantageously, this "swirling" inhalation airflow may induce rotation of the capsule to provide a uniform entrainment of a portion or a fraction of nicotine particles from the capsule over two or more, or five or more, or ten or more inhalations or "puffs" by a user.

Preferably a capsule is disposed within the capsule cavity of the inhaler article. The capsule preferably contains pharmaceutically active particles comprising nicotine. The pharmaceutically active particles may have a mass median aerodynamic diameter of about 5 micrometres or less, or in a range from about 0.5 micrometres to about 4 micrometres, or in a range from about 1 micrometres to about 3 micrometres.

The capsule may further contain a second population of flavour particles having a mass median aerodynamic diameter of about 20 micrometres or greater, or about 50 micrometres or greater, or in a range from about 50 to about 200 micrometres, or from about 50 to about 150 micrometres.

According to an aspect of the present invention, there is provided an inhaler system comprising an inhaler article as described herein and a holder configured to receive the inhaler article.

The holder may further comprise a piercing element removably engageable with the inhaler article to activate the capsule and wherein the twisted distal end element is configured to be pierced by the piercing element when activating the capsule.

According to an aspect of the present invention, there is provided a method of manufacturing a distal end element for an inhaler article, the method comprising: providing a distal end element material having a longitudinal axis, an outer surface, an element material distal end and an element material inner end, wherein the distal end element material comprises at least two grooves on the outer surface extending from the element material distal end to the element material inner end; and twisting at least one of the element material distal end and the element material inner end about the longitudinal axis such that the grooves are helically formed about the longitudinal axis to form a twisted distal end element.

Preferably the twisting step deforms the distal end element material so that the twisted form is maintained. The twisting step preferably rotates the element distal end at least 90 degrees or at least 180 degrees relative to the element inner end. Preferably, the twisting step deforms the distal end element permanently.

Advantageously, the distal end element may be easily and reliably formed. Advantageously, the distal end element may be easily and reliably assembled into the dry powder inhaler article. Advantageously, the inhaler article is in a form similar to a conventional cigarette. This may enable high speed assembly or manufacture of the inhaler article.

The inhaler article described herein may provide dry powder to the lungs at inhalation or air flow rates that are within conventional smoking regime inhalation or air flow rates. A user may take a plurality of inhalations or "puffs" where each "puff" delivers a fractional amount of dry powder contained within a capsule contained within the capsule cavity. This inhaler article may have a form similar to a conventional cigarette and may mimic the ritual of conventional smoking. This inhaler may be simple to manufacture and convenient to use by a user.

The inhaler article described herein may be combined with a piercing element or piercing device (that may form a portion of a holder) to deliver the nicotine particles from the capsule to a user. The piercing element or piercing device may be separated from or not form a portion of the inhaler article. A plurality of the inhaler articles may be combined with a piercing element or piercing device to form a kit.

Air flow management through the capsule cavity may cause the capsule to rotate during inhalation and consumption. The capsule contains nicotine particles comprising nicotine (also referred to as "nicotine powder" or "nicotine particles") and optionally particles comprising flavour (also referred to as "flavour particles). Rotation of the pierced capsule may suspend and aerosolize the nicotine particles released from the pierced capsule into the inhalation air moving through the inhaler article. The flavour particles may be larger than the nicotine particles and may assist in transporting the nicotine particles into the lungs of the user while the flavour particles preferentially remain in the mouth or buccal cavity of the user. The nicotine particles and optional flavour particles may be delivered with the inhaler article at inhalation or air flow rates that are within conventional smoking regime inhalation or air flow rates.

The term "porous" refers to a material containing pores. In particular porous refers to a non-woven fiber material that if formed of a fiber matrix defining pores. The porous material has a "resistance to draw" or "RTD" that is in a range of about 30 to about 90 millimetres (mm) water.

The phrase "resistance to draw" or "RTD" refers to the static pressure difference between the two ends of a specimen when it is traversed by an air flow under steady conditions in which the volumetric flow is 17.5 millilitres per second at the output end. The RTD of a specimen can be measured using the method set out in ISO Standard 6565:2002.

The term "nicotine" refers to nicotine and nicotine derivatives such as free-base nicotine, nicotine salts and the like.

The term "flavourant" or "flavour" refers to organoleptic compounds, compositions, or materials that alter and are intended to alter the taste or aroma characteristics of nicotine during consumption or inhalation thereof. The term "flavourant" or "flavour" preferably refers to compounds disclosed in the Flavor & Extract Manufacturers Association (FEMA) Flavor Ingredient Library and in particular in the GRAS Flavoring Substances publications 3 to 27, for example, see Hall, R.L. & Oser, B.L., Food Technology, February 1965 pg 151-197, and in the GRAS flavoring substances 27 S.M. Cohen et al., Food Technology Aug. 2015 pg. 40-59, and intervening GRAS Flavoring Substances publications 4 to 26. For the purpose of this disclosure, nicotine is not considered as a flavourant or flavour.

The terms "upstream" and "downstream" refer to relative positions of elements of the inhaler described in relation to the direction of inhalation air flow as it is drawn through the body of the inhaler from a distal end to the mouthpiece portion.

The terms "proximal" and "distal" are used to describe the relative positions of components, or portions of components, of the holder, inhaler article, or system. Inhaler articles, according to the invention have a proximal end which, in use, particles exit the proximal end of the inhaler article for delivery to a user and have an opposing distal end receiving incoming inhalation airflow to the inhaler article. The proximal end of the inhaler article may also be referred to as the mouth end.

As used herein, the singular forms "a", "an", and "the" encompass embodiments having plural referents, unless the content clearly dictates otherwise.

As used herein, "or" is generally employed in its sense including "and/or" unless the content clearly dictates otherwise. The term "and/or" means one or all of the listed elements or a combination of any two or more of the listed elements.

As used herein, "have", "having", "include", "including", "comprise", "comprising" or the like are used in their open ended sense, and generally mean "including, but not limited to". It will be understood that "consisting essentially of", "consisting of", and the like are subsumed in "comprising," and the like.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the disclosure, including the claims.

An inhaler article, according to the disclosure, includes a body extending along a longitudinal center axis from a mouthpiece end to a distal end. A capsule cavity is defined within the body. A distal end element is disposed at the distal end and extends to the capsule cavity. The distal end element includes an element distal end and an element inner end that is adjacent to the capsule cavity. The distal end element includes a continuous solid core portion and at least two helical grooves extending from the element distal end to the element inner end. The at least two helical grooves are parallel grooves that rotate about the continuous solid core portion along the longitudinal center axis from the element distal end to the element inner end. The at least two helical grooves may extend along an outer surface or periphery of the distal end element.

The body of the inhaler article, or the "inhaler body", may have any suitable shape. The body of the inhaler article, or "inhaler body" may resemble a smoking article or conventional cigarette in size and shape. The inhaler body may have an elongated cylindrical body extending along the longitudinal center axis of the inhaler article. In other words, the inhaler body may have a length that is substantially greater than the other dimensions of the inhaler body. The inhaler body may have a substantially uniform outer diameter along the length of the inhaler body. The inhaler body may have a substantially uniform inner diameter along the length of the inhaler body. The inhaler body may have any suitable transverse cross-sectional shape. For example, the transverse cross-section may be circular, elliptical, square or rectangular. The inhaler body may have a circular cross-section that may be uniform along the length of the inhaler body, forming an elongated cylindrical body. The inhaler body may be, or define, an outer wrapper of the inhaler article.

The inhaler body may have an outer diameter in a range from about 6 mm to about 10 mm, or from about 6 mm to about 9 mm, or about 6 mm to about 8 mm. The inhaler body may have a length (along the longitudinal axis) in a range from about 40 mm to about 100 mm, or from about 40 mm to about 80 mm, or about 40 mm to about 60 mm.

The inhaler body may be formed of a polymeric or cellulosic material, or any other suitable material. The inhaler body may be formed of a biodegradable material. The inhaler body may be formed of paperboard or cardboard. The inhaler body may have a uniform thickness along its length. The inhaler body may have a thickness in a range from about 1 mm to about 2 mm.

The inhaler body may form a unitary construction where the body extends continuously from the distal end element to the mouthpiece end. The distal end element, the capsule cavity (and capsule if present), and a porous support element (or filter) may be serially disposed within the inhaler body. In other words, the distal end element, the capsule cavity (and capsule if present), the porous support element (or filter) may be arranged end to end along the longitudinal axis of the inhaler body. The porous support element (or filter) may be disposed in a mouthpiece of the body. The mouthpiece air channel may extend from the porous support element (or filter) to the mouthpiece end. The porous support element (or filter) may extend from the capsule cavity to the mouthpiece end of the inhaler device.

The inhaler body may be formed of two or more portions. The two or more portions may be axially aligned in serial abutting relationship and joined together to form the inhaler body. A wrapper may be utilized to join the two or more portions together. The wrapper may be a biodegradable material. The wrapper may be a paper wrapper.

The distal end element may be disposed within the distal end of the body. The element distal end may be substantially aligned with the distal end of the body. The distal end element may define an upstream boundary of the capsule cavity.

The distal end element may be configured to induce a swirling air flow within the capsule cavity of the inhaler body. The distal end element may include helical grooves forming air channels or passageways for inlet air into the capsule cavity of the inhaler body at the element distal end and extending through the distal end element for outlet at the element inner end. The distal end element may induce rotational air flow or swirling air flow as the air flows through the helical grooves and through the capsule cavity. Air flow through the inhaler device preferably enters the inhaler device at the element distal end and flows along the longitudinal axis of the inhaler device to exit at the mouthpiece end at the proximal end of the inhaler device.

The distal end element may have a length between the element distal end and the element inner end that extends along the longitudinal axis (or longitudinal center axis) of the inhaler body. The distal end element may have any suitable length, such as between about 5 mm to about 15 mm. The distal end element may have a length of in the range of about 7 mm to about 12 mm.

The distal end element (including the outer periphery) may substantially fill the inner diameter of the inhaler body. The distal end element may have an outer diameter in a range from about 7 mm to about 7.5 mm, or from about 7.1 mm to about 7.2 mm. The distal end element may be sized and shaped to fit within and against the inner surface of the body. The distal end element may have an outer diameter sufficient to form a friction fit or interference fit with the inner surface of the inhaler body.

The grooves of the distal end element may extend along the inner surface of the body. The grooves may be longitudinally closed along the outer periphery of the distal end element by the inner surface of the body. The grooves may be defined along an outer surface, or outer periphery, of the distal end element. The grooves may be open (not closed) along the outer surface of the distal end element.

The grooves of the distal end element may extend from the outer diameter of the core to an outer periphery of the distal end element. In other words, the outer diameter of the core may define an inner periphery of the grooves. The distal end element may include at least two helical grooves extending non-parallel to the longitudinal axis (or longitudinal center axis) of the inhaler device. The grooves may be continuously non-parallel with the longitudinal axis of the inhaler device along an entire length of the distal end element. The at least two grooves may extend curve-linearly or helically along the longitudinal length of the distal end element. The at least two grooves extend in parallel to one another. The at least two grooves may preferably include three grooves, and may preferably include four grooves, and may preferably include four or more grooves. The at least two grooves preferably extend along the outer periphery of the distal end element. The at least two grooves may be radially offset from one another. The at least two grooves may be equally radially spaced around the outer periphery of distal end element.

The grooves may each have generally triangular cross-section, having sides defined by generally rectangular extensions of the distal end element radially extending from the core at an outer periphery. In other words, each of the grooves extends between adjacent extensions exterior of the core, at the outer periphery, of the distal end element. The at least two grooves may have any suitable or useful shape, such as a semi-circular cross-section, a generally rectangular or square cross-section, or other suitable cross-sectional shape to provide a swirling airflow to the capsule cavity. The grooves may define a total air inlet cross-sectional area in a range from about 1 square mm to about 2 square mm, or about 1.4 square mm to about 1.8 square mm, or about 1.6 square mm. The term "total air inlet cross-sectional area" refers to the sum of the cross-sectional areas of all of the air inlets providing inhalation air into the capsule cavity of the inhaler article.

The core of the distal end element may be centrally disposed along the longitudinal axis of the distal end element. The core and the grooves may each extend continuously from the element distal end to the element inner end. The core may be formed as a continuous body of material. The core may be formed as a continuous solid body of material. Preferably the core forms a continuous solid core that extends an entire longitudinal length of the distal end element.

The core may extend along the longitudinal center axis of the article. The core may have a circular cross-section. The core may have a diameter in a range from about 50% to about 75% of the diameter of the distal end element. The core may have a diameter in the range of about 4 mm to about 6 mm, or from about 4.7 mm to about 5 mm, or from about 4.8 mm to about 4.9 mm.

The distal end element may be formed of a fibrous material. The distal end element may be formed of a porous material. The distal end element may be formed of biodegradable or bio-based materials. Some biodegradable or bio-based materials that may be used to form the distal end element include polyvinyl alcohol (PVOH), polylactic acid (PLA), or polyhydroxybutyrate (PHB).

The distal end element may be porous or fibrous enough to allow passage of piercing element to allow for mechanical puncturing of the capsule within the capsule cavity. The distal end element may heal or fill in any piercing aperture formed by the piercing element once the piercing element is withdrawn from the distal end element.

The distal end element may be formed of a cellulose material. The distal end element may be formed of a cellulose acetate material. The distal end element may be formed of a polylactic acid material.

The distal end element may be formed by twisting a distal end element material having at least two linear grooves about a longitudinal center axis such that the grooves become curvilinear. The distal end element may be formed with an extrusion process such that the distal end element includes linear grooves that are subsequently twisted into a helical formation. The twisting process may rotate the element distal end at least 90 degrees relative to the element inner end. The twisting process may rotate the element distal end at least 180 degrees relative to the element inner end. The distal end element material may be twisted such that the core continuously extends along the longitudinal center axis and the grooves are moved into a parallel helical formation.

The twisting process may deform the distal end element material such that the grooves are helically formed about the longitudinal axis to form a twisted distal end element. The twisting process may deform the distal end element to maintain the twisted form.

The distal end element may be sprayed or otherwise coated to permanently affix the distal end element in the twisted form. The distal end element may be formed with an extrusion process wherein the extrusion die-head is rotated to form the grooves in a helical pattern on the outer periphery as the distal end element is extruded to form the grooves with a desired pitch and depth. The distal end element may be cut to the appropriate length prior to insertion into the body of the inhaler.

The capsule cavity may be axially aligned and in downstream serial arrangement with the distal end element. The distal end element may form an upstream or distal end or boundary of the capsule cavity. The capsule cavity may define a cylindrical space configured to contain a capsule. The capsule cavity may define a space configured to receive a capsule having an obround or rounded rectangular shape. The capsule cavity may have a substantially uniform or uniform diameter along the length of the capsule cavity. The capsule cavity may have a circular transverse cross-section along the length of the capsule cavity. The capsule cavity may have a cylindrical shape. The configuration of the capsule cavity relative to the capsule may allow the capsule to rotate with stability within the capsule cavity. The longitudinal axis of the capsule may rotate with stability about the longitudinal axis of the inhaler body during inhalation.

Stable rotation refers to the longitudinal axis of the inhaler body being substantially parallel with the axis of rotation of the capsule. Stable rotation may refer to the absence of procession of the rotating capsule. Preferably the longitudinal axis of the inhaler body may be substantially coextensive with the axis of rotation of the capsule. Stable rotation of the capsule may provide a uniform entrainment of a portion of nicotine particles from the capsule over two or more, or five or more, or ten or more "puffs" by a consumer.

The capsule cavity may have a fixed cavity length bounded on an upstream or inner end of the distal end element and bounded on the downstream end by a porous support element or filter. The capsule cavity may have a cavity length of about at least about 110% to less than about 200% of a length of the capsule contained therein, or from about 120% to about 130% of the capsule length, or about 125% of the capsule length. The cavity length may be in a range from about 15 mm to about 25 mm and the capsule length may be in a range from about 14 to about 18 mm, or the cavity length may be about 20 mm and the capsule length may be about 16 mm. The cavity length may be about 20 mm. The capsule cavity and the distal end element may have a combined length from about 25 mm to about 35 mm, or the combined length may be about 27 mm to about 32 mm.

The capsule cavity has a cavity inner diameter, orthogonal to the longitudinal axis, and the capsule has a capsule outer diameter. The capsule outer diameter may be in a range from about 80% to about 99% of the cavity inner diameter, or capsule outer diameter may be in a range from about 85% to about 95% of the cavity inner diameter, or capsule outer diameter may be about 90% of the cavity inner diameter. The capsule outer diameter may be in a range from about 5.4 mm to about 6.4 mm and the cavity inner diameter may be in a range from about 6 mm to about 7 mm.

An insert body may be included within the capsule cavity. The insert body may define a cylindrical space configured to contain a capsule. The insert body may provide additional rigidity to the body along the capsule cavity. The insert body may have a substantially uniform or uniform diameter along the length of the capsule cavity. The insert body may have a length of about at least about 110% to less than about 200% of a length of the capsule contained therein, or from about 120% to about 130% of the capsule length, or about 125% of the capsule length. The insert body may be formed of the same or different material as the body. Preferably the insert body is formed of paperboard or cardboard.

The capsule cavity may be bounded on an upstream end by the by the distal end element and bounded on a downstream end or mouthpiece side by the porous support element or filter. The distal end element and porous support element may cooperate to contain the capsule longitudinally within the capsule cavity. The distal end element and the porous support element may each fill the inner diameter of the elongated inhaler body. The porous support element may allow air flow to exhibit a uniform airflow along the cross-section of the elongated inhaler body through the porous support element. The porous support element may function as a filter or diffuser to reduce turbulence effects or edge effects and ensure or maintain the desired air flow pattern through the capsule cavity. The porous support element may support a capsule inside the capsule cavity during activation of the capsule, such as by providing a support for the capsule as a piercing element is received in the inhaler article at the distal end and pierces the capsule to activate the capsule.

A capsule may be sealed within the inhaler article prior to consumption. For transport and storage, the inhaler article may be contained within a sealed or airtight container or bag. The inhaler article may include one or more peelable seal layers to cover the one or more air inlet channels at the distal end or the air outlet at the mouthpiece end of the inhaler article. This may ensure the inhaler articles maintain appropriate hygiene and freshness or may prevent the capsule from drying out and becoming hard or friable.

The capsule may rotate about its longitudinal or central longitudinal axis when air is drawn through the inhaler article. The capsule may be formed of an airtight material that substantially contains the particles inside the capsule. The capsule may be configured to be pierced or punctured by a piercing element when the capsule is within the capsule cavity. The piercing element may be separate or combined with the inhaler article. The capsule may be formed of any suitable material. The capsule may be formed of a metallic or polymeric material that serves to keep contaminants out of the capsule but may be pierced or punctured by a piercing element prior to consumption to enable the release of the nicotine particles from within the capsule. The capsule may be formed of a polymer material. The polymer material may be hydroxypropylmethylcellulose (HPMC). The capsule may be any suitable size. The capsule may be a size 1 to size 4 capsule, or a size 3 capsule, or a size 3 capsule.

An inhaler system includes an inhaler article as described herein and a holder configured to receive the inhaler article. The holder may include a piercing element to activate or pierce a capsule that is disposed within the capsule cavity of the inhaler device.

The inhaler system may comprise a separate piercing element, such as a metal or rigid needle. The piercing element may form a single aperture through the capsule received in the capsule cavity. The piercing element may be configured to pass through the continuous solid core portion of the distal end element and into the capsule cavity. The continuous solid core portion of the distal end element may be formed of a porous material, as described above. The distal end element may be substantially self-sealing after the piercing element has been withdrawn from the inhaler article.

The capsule contains nicotine particles comprising nicotine (also referred to as "nicotine powder" or "nicotine particles") and optionally particles comprising flavour (also referred to as "flavour particles). The capsule may contain a predetermined amount of nicotine particles and optional flavour particles. The capsule may contain enough nicotine particles to provide at least 2 inhalations or "puffs", or at least about 5 inhalations or "puffs", or at least about 10 inhalations or "puffs". The capsule may contain enough nicotine particles to provide from about 5 to about 50 inhalations or "puffs", or from about 10 to about 30 inhalations or "puffs". Each inhalation or "puff" may deliver from about 0.1 mg to about 3 mg of nicotine particles to the lungs of the user or from about 0.2 mg to about 2 mg of nicotine particles to the lungs of the user or about 1 mg of nicotine particles to the lungs of the user.

The nicotine particles may have any useful concentration of nicotine based on the particular formulation employed. The nicotine particles may have at least about 1 %wt nicotine up to about 30%wt nicotine, or from about 2%wt to about 25%wt nicotine, or from about 3%wt to about 20%wt nicotine, or from about 4%wt to about 15%wt nicotine, or from about 5%wt to about 13%wt nicotine. Preferably, about 50 to about 150 micrograms of nicotine may be delivered to the lungs of the user with each inhalation or "puff".

The capsule may hold or contain at least about 5 mg of nicotine particles or at least about 10 mg of nicotine particles. The capsule may hold or contain less than about 900 mg of nicotine particles, or less than about 300 mg of nicotine particles, or less than 150 mg of nicotine particles. The capsule may hold or contain from about 5 mg to about 300 mg of nicotine particles or from about 10 mg to about 200 mg of nicotine particles.

When flavour particles are blended or combined with the nicotine particles within the capsule, the flavour particles may be present in an amount that provides the desired flavour to each inhalation or "puff" delivered to the user.

The nicotine particles may have any useful size distribution for inhalation delivery preferentially into the lungs of a user. The capsule may include particles other than the nicotine particles. The nicotine particles and the other particles may form a powder system.

The capsule may hold or contain at least about 5 mg of a dry powder (also referred to as a powder system) or at least about 10 mg of a dry powder. The capsule may hold or contain less than about 900 mg of a dry powder, or less than about 300 mg of a dry powder, or less than about 150 mg of a dry powder. The capsule may hold or contain from about 5 mg to about 300 mg of a dry powder, or from about 10 mg to about 200 mg of a dry powder, or from about 25 mg to about 100 mg of a dry powder.

The dry powder or powder system may have at least about 40%, or at least about 60%, or at least about 80%, by weight of the powder system comprised in nicotine particles having a particle size of about 5 micrometres or less, or in a range from about 1 micrometre to about 5 micrometres.

The particles comprising nicotine may have a mass median aerodynamic diameter of about 5 micrometres or less, or in a range from about 0.5 micrometres to about 4 micrometres, or in a range from about 1 micrometres to about 3 micrometres or in a range from about 1.5 micrometres to about 2.5 micrometres. The mass median aerodynamic diameter is preferably measured with a cascade impactor.

The particles comprising flavour may have a mass median aerodynamic diameter of about 20 micrometres or greater, or about 50 micrometres or greater, or in a range from about 50 to about 200 micrometres, or from about 50 to about 150 micrometres. The mass median aerodynamic diameter is preferably measured with a cascade impactor.

The dry powder may have a mean diameter of about 60 micrometres or less, or in a range from about 1 micrometres to about 40 micrometres, or in a range from about 1.5 micrometres to about 25 micrometres. The mean diameter refers to the mean diameter per mass and is preferably measured by laser diffraction, laser diffusion or an electronic microscope.

Nicotine in the powder system or nicotine particles may be a pharmaceutically acceptable free-base nicotine, or nicotine salt or nicotine salt hydrate. Useful nicotine salts or nicotine salt hydrates include nicotine pyruvate, nicotine citrate, nicotine aspartate, nicotine lactate, nicotine bitartrate, nicotine salicylate, nicotine fumarate, nicotine mono-pyruvate, nicotine glutamate or nicotine hydrochloride, for example. The compound combining with nicotine to form the salt or salt hydrate may be chosen based on its expected pharmacological effect.

The nicotine particles preferably include an amino acid. Preferably the amino acid may be leucine such as L-leucine. Providing an amino acid such as L-leucine with the particles comprising nicotine, may reduce adhesion forces of the particles comprising nicotine and may reduce attraction between nicotine particles and thus reduce agglomeration of nicotine particles. Similarly, adhesion forces to particles comprising flavour may also be reduced thus agglomeration of nicotine particles with flavour particles is also reduced. The powder system described herein thus may be a free-flowing material and possess a stable relative particle size of each powder component even when the nicotine particles and the flavour particles are combined.

Preferably, the nicotine may be a surface modified nicotine salt where the nicotine salt particle comprises a coated or composite particle. A preferred coating or composite material may be L-leucine. One particularly useful nicotine particle may be nicotine bitartrate with L-leucine.

The powder system may include a population of flavour particles. The flavour particles may have any useful size distribution for inhalation delivery selectively into the mouth or buccal cavity of a user.

The powder system may have at least about 40%, or at least about 60%, or at least about 80%, by weight of the population of flavour particles of the powder system comprised in particles having a particle size of about 20 micrometres or greater. The powder system may have at least about 40% or at least about 60%, or at least about 80%, by weight of the population of flavour particles of the powder system comprised in particles having a particle size of about 50 micrometres or greater. The powder system may have at least about 40% or at least about 60%, or at least about 80%, by weight of the population of flavour particles of the powder system comprised in particles having a particle size in a range from about 50 micrometres to about 150 micrometres.

The particles comprising flavour may include a compound to reduce adhesion forces or surface energy and resulting agglomeration. The flavour particle may be surface modified with an adhesion reducing compound to form a coated flavour particle. One preferred adhesion reducing compound may be magnesium stearate. Providing an adhesion reducing compound such as magnesium stearate with the flavour particle, especially coating the flavour particle, may reduce adhesion forces of the particles comprising flavour and may reduce attraction between flavour particles and thus reduce agglomeration of flavour particles. Thus, agglomeration of flavour particles with nicotine particles may also be reduced. The powder system described herein thus may possess a stable relative particle size of the particles comprising nicotine and the particles comprising flavour even when the nicotine particles and the flavour particles are combined. The powder system preferably may be free flowing.

Conventional formulations for dry powder inhalation contain carrier particles that serve to increase the fluidization of the active particles since the active particles may be too small to be influenced by simple airflow though the inhaler. The powder system may comprise carrier particles. These carrier particles may be a saccharide such as lactose or mannitol that may have a particle size greater than about 50 micrometres. The carrier particles may be utilized to improve dose uniformity by acting as a diluent or bulking agent in a formulation.

The powder system utilized with the nicotine powder delivery system described herein may be carrier-free or substantially free of a saccharide such as lactose or mannitol. Being carrier-free or substantially free of a saccharide such as lactose or mannitol may allow the nicotine and to be inhaled and delivered to the user's lungs at inhalation or airflow rates that are similar to typical smoking regime inhalation or airflow rates.

The nicotine particles and a flavour may be combined in a single capsule. As described above, the nicotine particles and a flavour may each have reduced adhesion forces that result in a stable particle formulation where the particle size of each component does not substantially change when combined. Alternatively, the powder system includes nicotine particles contained within a single capsule and the flavour particles contained within a second capsule.

The nicotine particles and flavour particles may be combined in any useful relative amount so that the flavour particles are detected by the user when consumed with the nicotine particles. Preferably the nicotine particles and a flavour particles form at least about 90%wt or at least about 95%wt or at least about 99%wt or 100%wt of the total weight of the powder system.

The inhaler and inhaler system may be less complex and have a simplified airflow path as compared to conventional dry powder inhalers. Advantageously, rotation of the capsule within the inhaler body aerosolizes the nicotine particles or powder system and may assist in maintaining a free-flowing powder. Thus, the inhaler article may not require the elevated inhalation rates typically utilized by conventional inhalers to deliver the nicotine particles described above deep into the lungs.

The inhaler article may use a flow rate of less than about 5 L/min or less than about 3 L/min or less than about 2 L/min or about 1.6 L/min. Preferably, the flow rate may be in a range from about 1 L/min to about 3 L/min or from about 1.5 L/min to about 2.5 L/min. Preferably, the inhalation rate or flow rate may be similar to that of Health Canada smoking regime, that is, about 1.6 L/min.

The inhaler system may be used by a consumer like smoking a conventional cigarette or vaping an electronic cigarette. Such smoking or vaping may be characterized by two steps: a first step during which a small volume containing the full amount of nicotine desired by the consumer is drawn into the mouth cavity, followed by a second step during which this small volume comprising the aerosol comprising the desired amount of nicotine is further diluted by fresh air and drawn deeper into the lungs. Both steps are controlled by the consumer. During the first inhalation step the consumer may determine the amount of nicotine to be inhaled. During the second step, the consumer may determine the volume for diluting the first volume to be drawn deeper into the lungs, maximizing the concentration of active agent delivered to the airway epithelial surface. This smoking mechanism is sometimes called "puff-inhale-exhale".

The dry powder utilized with the dry powder inhaler of the invention may eliminate or substantially reduce any exhalation of pharmaceutically active particles during the "exhale" phase. Preferably nearly all, or at least about 99% or at least about 95% or at least 90% of the pharmaceutically active particle has a particle size that is delivered to the lungs but are not small enough to be exhaled by tidal breathing. This pharmaceutically active particle size may be in a range from about 0.75 micrometres to about 5 micrometres, or from 0.8 micrometres to about 3 micrometres, or from 0.8 micrometres to about 2 micrometres.

The inhaler article described herein may be combined with an holder for activating the inhaler article by piercing the capsule, providing reliable activation of the capsule (by puncturing the capsule with the piercing element of the holder) within inhaler article, and releasing the particles contained inside the capsule and enabling the article to deliver the particles to a consumer. The holder is separate from the inhaler article, but the consumer may utilize both the inhaler article and the holder while consuming the particles released within the inhaler article. A plurality of these inhaler articles may be combined with a holder to form a system or kit. A single holder may be utilized on 10 or more, or 25 or more, or 50 or more, or 100 or more, inhaler articles to activate (puncture or pierce) a capsule contained within each inhaler article and provide reliable activation and optionally, a visual indication (marking), for each inhaler article of the activation of the inhaler article.

An inhaler system may include the inhaler and a holder. The holder for the inhaler may include a housing. The holder for the inhaler may include a piercing element. The holder for the inhaler may include a sleeve configured to receive the inhaler article and may be movable within the holder.

The holder may retain the activated inhaler article and a user may grasp the holder and consume particles within the inhaler article. The housing has a housing outer surface and a housing inner surface. The housing inner surface defines an inhaler article cavity. The housing extends along a housing longitudinal axis from a distal end to an open proximal end a housing length. The housing open proximal end is configured to receive the distal end of an inhaler into the inhaler article cavity. The piercing element is fixed to and extends from the housing inner surface, into the inhaler article cavity along a piercing element longitudinal axis a piercing element length. The piercing element is recessed from the open proximal end a recessed distance. The sleeve is disposed within the inhaler article cavity and configured to retain an inhaler. The sleeve is movable along the housing longitudinal axis.

The holder may include a spring element configured to bias the sleeve toward the open proximal end of the housing, and between a relaxed and compressed position. The spring element may be contained within the inhaler article cavity of the holder and be compressed as the movable sleeve and inhaler article move toward the piercing element. The spring element may be between the sleeve and distal end of the housing and contact the sleeve and distal end of the housing. The spring element may be between the distal end of the sleeve and the distal end of the housing. The spring element may contact the distal end of the sleeve and the distal end of the housing. The spring element may be disposed about the piercing element. The spring element may be co-axial with the piercing element. The spring element may be a conical spring.

The spring element biases the inhaler article away from the piercing element. In use, a user may insert an inhaler article into the inhaler article cavity of the holder. By doing this, the spring may be compressed allowing the inhaler article to move towards the distal end of the inhaler article cavity. Eventually, the piercing element may penetrate a capsule disposed within the inhaler article. Once this happens, the user may release the inhaler article, allowing the spring to bias the inhaler article towards the proximal end of the inhaler article cavity and away from the piercing element. The user may then inhale on the proximal end of the inhaler article.

The sleeve may define a first air inlet zone comprising at least one air aperture through the sleeve. The first air inlet zone is proximate to a proximal end of the sleeve. The first air inlet zone is configured to allow air to flow from an inside of the sleeve to an airflow channel formed between the sleeve and the housing inner surface. The sleeve may comprise a second air inlet zone comprising at least one air aperture through the sleeve. The second air inlet zone is proximate to a distal end of the sleeve. The second air inlet zone is configured to allow air to flow from the airflow channel to an inside of the sleeve.

The sleeve defines an inner cavity and a portion of the inner cavity may have a reduced internal diameter relative to remaining portions of the inner cavity. Preferably, a portion of the inner cavity may have an internal diameter which is the same as or less than the outer diameter of an inhaler article.

Referring now to the drawings, in which some aspects of the present invention are illustrated.
FIG. 1 is a cross-sectional diagram of an inhaler system including illustrative an inhaler article along a longitudinal axis.
FIG. 2 is a cross-sectional diagram of an inhaler system including another illustrative inhaler article along a longitudinal axis.
FIG. 3 is a side schematic diagram of an illustrative distal end element useful in the inhaler articles illustrated in FIGS. 1 and 2.
FIG. 4A-4C are transverse cross-sectional, proximal and distal end diagrams of the distal end element illustrated in FIG. 3.
FIG. 5A is a side elevation schematic diagram of an illustrative flat holder useful with an inhaler article.
FIG. 5B is a top elevation schematic diagram of the illustrative flat holder of FIG. 5A.
FIG. 5C is a cross-sectional schematic diagram of the illustrative flat holder of FIG. 5B taken along line A-A.

The schematic drawings are not necessarily to scale and are presented for purposes of illustration and not limitation. The drawings depict one or more aspects described in this disclosure. However, it will be understood that other aspects not depicted in the drawing fall within the scope and spirit of this disclosure.

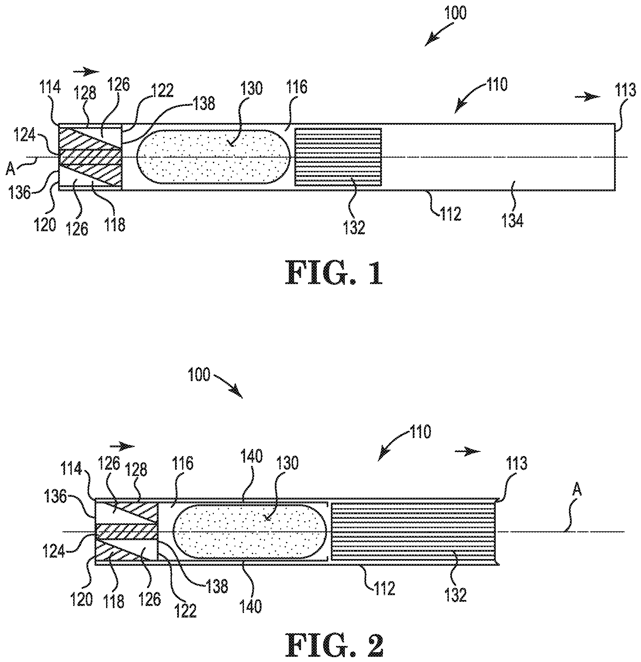

FIGS. 1 and 2 illustrate exemplary inhaler systems 100 including inhaler articles 110. The inhaler article 110 may include a capsule 130 disposed within inhaler article 110 to comprise the inhaler system 100.

The inhaler article 110 includes a body 112 extending along a longitudinal center axis "A" from a mouthpiece end 113 to a distal end 114. The mouthpiece end 113 forms a downstream end and distal end 114 forms an upstream end of the inhaler article 110. In other words, the mouthpiece end 113 is downstream from the distal end, as indicated with arrows. A capsule cavity 116 is defined within the body 112. A distal end element 118 is disposed at the distal end 114 and extends to the capsule cavity 116.

The distal end element 118 includes an element distal end 120 and an element inner end 122 that is adjacent to the capsule cavity 116. The distal end element 118 includes a solid core portion 124 and at least two helical grooves 126 extending from the element distal end 120 to the element inner end 122. The at least two helical grooves 126 are parallel grooves that extend about the longitudinal center axis "A" from the element distal end 120 to the element inner end 122. The at least two helical grooves 126 extend along an outer periphery 128 of the distal end element 118.

The distal end element 118, the capsule cavity 116 (and capsule 130 if present), and a porous support element 132 may be axially aligned and serially disposed within the body 112. The distal end element 118 may form an upstream or distal end or boundary of the capsule cavity 116. The capsule cavity 116 may define a space configured to contain the capsule 130. The capsule cavity 116 may have a fixed cavity length bounded on the element inner end 122 of the distal end element 118 and bounded on the downstream end by a porous support element 132. As illustrated in FIG. 1, the porous support element 132 may be disposed within a mouthpiece air channel portion 134 of the body 110. The mouthpiece air channel portion 134 may extend beyond the porous support element 132 to the mouthpiece end 112.

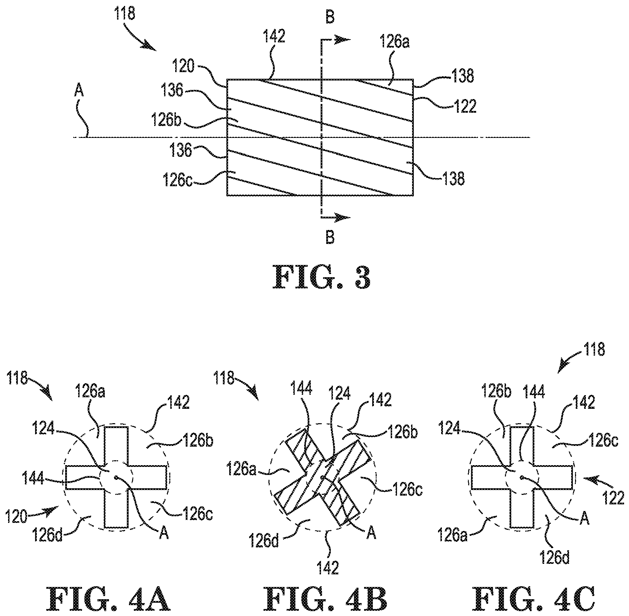

The distal end element 118 may be disposed within the distal end 114 of the body 112. The element distal end 120 may be substantially aligned with the distal end 114 of the body 112. The distal end element 118 may include at least two helical grooves 126 to form air channels or passageways extending through the distal end element 118 from the element distal end 120 to the element inner end 122. With additional reference to FIG. 3, each of the at least two helical grooves 126 includes an air inlet 136 at the element distal end 120 and an air outlet 138 at the element inner end 122. The at least two helical grooves 126 may extend non-parallel to the longitudinal center axis "A" of the inhaler article. The helical grooves 126 may be continuously non-parallel with the longitudinal center axis "A" of the inhaler article 110 along an entire length of the distal end element 118.

As illustrated in FIG. 2, the inhaler article 110 may include an insert body 140 defining the capsule cavity 116 to contain the capsule 130 and provide support and rigidity to the body 112 along the capsule cavity 116. The porous support element 132 extends from the capsule cavity 116 to the mouthpiece end 113 of the inhaler article 110.

FIGS. 3 and 4A-4C illustrate views of the distal end element 118 of the inhaler article 110 of FIGS. 1 and 2. FIG. 3 is a side view of the distal end element 118. FIGS. 4A-4C are cross-sectional, proximal and distal end views of the distal end element 118. FIG. 4B is taken along line B-B. The distal end element 118 comprises the solid core portion 124 extending along the longitudinal center axis "A". The core portion 124 and the helical grooves 126a-126d may each extend continuously from the element distal end 120 to the element inner end 122. FIGS. 4A-4C illustrate the distal end element 118 comprising four helical grooves 126a-126d extending along the outer surface 142, or outer periphery, of the distal end element 118. The helical grooves 126a-126d may be open (not closed) along the outer periphery 142 of the distal end element 118. The helical grooves 126a-126d of the distal end element 118 may extend from the outer diameter 144 of the core portion 124 to an outer periphery 142 of the distal end element 118. The helical grooves 126a-126d may extend curve-linearly along the longitudinal length of the distal end element 118 between the element distal end 120 and the element inner end 122. The helical grooves 126a-126d extend in parallel to one another. The helical grooves 126a-126d are radially offset from one another from the element distal end 120 to the element inner end 122. The parallel helical grooves 126a-126d may rotate at least 90 degrees from the element distal end 120 to the element inner end 122. The helical grooves 126a-126d may be equally radially spaced around the outer periphery 142 of element 118.

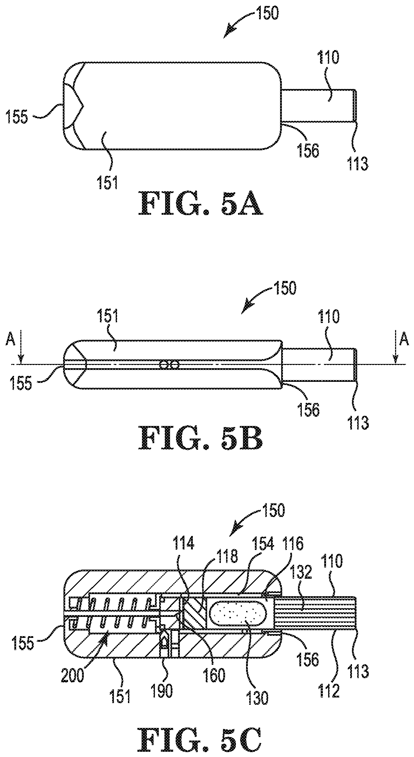

With reference to FIGS. 5A-5C, an inhaler system includes the inhaler article 110 and a holder 150. The inhaler article 110 comprises the body 112 defining an inhaler outer surface. The inhaler article 110 may be similar to either exemplary inhaler article 110 of FIG. 1 or FIG. 2. The body 112 extends along an inhaler longitudinal axis from mouthpiece, or proximal end 113 to a distal end 114 a body length.

The holder 150 for the inhaler article 110 includes, a housing 151 and a piercing element 160. The holder 150 may also include a marking element 190. The housing 151 has a housing outer surface and a housing inner surface. The housing inner surface defines an inhaler article cavity 154 configured to receive the inhaler article 110. The housing 151 extends along a housing longitudinal axis from a distal end 155 to an open proximal end 156 defining a housing length. The open proximal end 156 is configured to receive the distal end 114 of an inhaler article 110 into the inhaler article cavity 154. A piercing element 160 is fixed to and extends from the housing inner surface, into the inhaler article cavity 154 along a piercing element longitudinal axis a piercing element length. The piercing element 160 is recessed from the open proximal end 156 by a recessed distance. The piercing element 160 is configured to be movable to pierce the distal end element 118 and the capsule 130 housed within the capsule cavity 116. A spring element 200 may bias the inhaler article 110 away from the piercing element 160.

The exemplary embodiments described above are not limiting. Other embodiments consistent with the exemplary embodiments described above will be apparent to those skilled in the art.

## Claims

1. An inhaler article (110) comprising:
a body (112) extending along a longitudinal center axis (A) from a mouthpiece end (113) to a distal end (114);
a capsule cavity (116) defined within the body; and
a distal end element (118) disposed at the distal end of the inhaler article and extending to the capsule cavity, the distal end element comprising an element distal end (120), an element inner end (122), a solid core portion (124), and at least two grooves (126), wherein the solid core portion comprises a central portion of the distal end element, wherein the central portion is solid and extends along the longitudinal center axis from the element distal end to the element inner end, wherein the at least two grooves are helical grooves that rotate about the solid core portion along the longitudinal center axis from the element distal end to the element inner end, and wherein the at least two helical grooves extend along an outer surface (128) of the distal end element.

2. The inhaler article (110) according to claim 1, wherein the distal end element (118) is formed of a biodegradable material.

3. The inhaler article (110) according to any preceding claim, wherein the distal end element (118) comprises a fibrous material.

4. The inhaler article (110) according to any preceding claim, wherein the distal end element (118) is formed of a porous material.

5. The inhaler article (110) according to any preceding claim, wherein the distal end element (118) is formed of a cellulose material.

6. The inhaler article (110) according to any preceding claim, wherein the distal end element (118) is formed of a cellulose acetate material.

7. The inhaler article (110) according to any one of claims 1 to 4, wherein the distal end element (118) is formed of polylactic acid material.

8. The inhaler article (110) according to any preceding claim, wherein the grooves (126) rotate at least 90 degrees from the element distal end (120) to the element inner end (122).

9. The inhaler article (110) according to any preceding claim, wherein the element distal end (120) is substantially aligned with the distal end (114).

10. The inhaler article (110) according to any one of the preceding claims, wherein a capsule (130) is disposed within the capsule cavity (116) of the inhaler article.

11. The inhaler article (110) according to claim 10, wherein the capsule (130) contains pharmaceutically active particles comprising nicotine, the pharmaceutically active particles having a mass median aerodynamic diameter of about 5 micrometres or less, or in a range from about 0.5 micrometres to about 4 micrometres, or in a range from about 1 micrometre to about 3 micrometres.

12. An inhaler system comprising:
the inhaler article (110) according to any preceding claim; and
a holder (150) configured to receive the inhaler article.

13. A method of manufacturing a distal end element (118) for an inhaler article (110), the method comprising the steps of:
providing a distal end element material having a longitudinal axis (A), an outer surface (142), an element material distal end (120) and an element material inner end (122), wherein the distal end element material comprises at least two grooves (126) on the outer surface extending from the element material distal end to the element material inner end_and a solid core portion (124) comprising a central portion of the distal end element and extending along the longitudinal center axis from the element distal end to the element inner end, wherein the central portion is solid; and
twisting at least one of the element material distal end and the element material inner end about the longitudinal axis such that the grooves are helically formed about the longitudinal axis to form a twisted distal end element.

14. The method of claim 13, wherein the twisting step deforms the distal end element material so that the twisted distal end element (118) form is maintained.

15. The method of claim 13 or 14, wherein the twisting step comprises rotating the distal end element material distal end (120) at least 90 degrees relative to the distal end element material inner end (122).

## Patentansprüche

1. Inhalatorartikel (110), umfassend:
einen sich entlang einer Längsmittelachse (A) von einem Mundstückende (113) zu einem distalen Ende (114) erstreckenden Körper (112);
einen innerhalb des Körpers definierten Kapselhohlraum (116); und
ein an dem distalen Ende des Inhalatorartikels angeordnetes und sich zu dem Kapselhohlraum erstreckendes distales Endelement (118), wobei das distale Endelement ein distales Ende des Elements (120), ein inneres Ende des Elements (122), einen soliden Kernabschnitt (124) und wenigstens zwei Nuten (126) aufweist, wobei der solide Kernabschnitt einen zentralen Abschnitt des distalen Endelements umfasst, wobei der zentrale Abschnitt solide ist und sich entlang der Längsmittelachse von dem distalen Ende des Elements zu dem inneren Ende des Elements erstreckt, wobei die wenigstens zwei Nuten schraubenförmige Nuten sind, die sich um den soliden Kernabschnitt entlang der Längsmittelachse von dem distalen Ende des Elements zu dem inneren Ende des Elements drehen, und wobei sich die wenigstens zwei schraubenförmigen Nuten entlang einer Außenfläche (128) des distalen Endelements erstrecken.

2. Inhalatorartikel (110) nach Anspruch 1, wobei das distale Endelement (118) aus einem biologisch abbaubaren Material gebildet ist.

3. Inhalatorartikel (110) nach einem beliebigen vorhergehenden Anspruch, wobei das distale Endelement (118) ein Fasermaterial aufweist.

4. Inhalatorartikel (110) nach einem beliebigen vorhergehenden Anspruch, wobei das distale Endelement (118) aus einem porösen Material gebildet ist.

5. Inhalatorartikel (110) nach einem beliebigen vorhergehenden Anspruch, wobei das distale Endelement (118) aus einem Cellulosematerial gebildet ist.

6. Inhalatorartikel (110) nach einem beliebigen vorhergehenden Anspruch, wobei das distale Endelement (118) aus einem Celluloseacetatmaterial gebildet ist.

7. Inhalatorartikel (110) nach einem beliebigen der Ansprüche 1 bis 4, wobei das distale Endelement (118) aus einem Polymilchsäurematerial gebildet ist.

8. Inhalatorartikel (110) nach einem beliebigen vorhergehenden Anspruch, wobei sich die Nuten (126) um wenigstens 90 Grad von dem distalen Elementende (120) zu dem inneren Ende des Elements (122) drehen.

9. Inhalatorartikel (110) nach einem beliebigen vorhergehenden Anspruch, wobei das distale Elementende (120) im Wesentlichen mit dem distalen Ende (114) ausgerichtet ist.

10. Inhalatorartikel (110) nach einem beliebigen der vorhergehenden Ansprüche, wobei eine Kapsel (130) innerhalb des Kapselhohlraums (116) des Inhalatorartikels angeordnet ist.

11. Inhalatorartikel (110) nach Anspruch 10, wobei die Kapsel (130) pharmazeutisch aktive Partikel enthält, die Nikotin umfassen, wobei die pharmazeutisch wirksamen Partikel einen mittleren aerodynamischen Massendurchmesser von etwa 5 Mikrometern oder weniger oder in einem Bereich von etwa 0,5 Mikrometern bis etwa 4 Mikrometern oder in einem Bereich von etwa 1 Mikrometer bis etwa 3 Mikrometern aufweisen.

12. Inhalatorsystem, aufweisend:
einen Inhalatorartikel (110) nach einem beliebigen vorhergehenden Anspruch; und
einen zum Aufnehmen des Inhalatorartikels ausgelegten Halter (150).

13. Verfahren zum Herstellen eines distalen Endelements (118) für einen Inhalatorartikel (110), das Verfahren umfassend die Schritte:
Vorsehen eines distalen Endelementmaterials, aufweisend eine Längsachse (A), eine Außenfläche (142), ein distales Ende des Elementmaterials (120) und ein inneres Ende des
Elementmaterials (122), wobei das distale Endelementmaterial wenigstens zwei Nuten (126) auf der Außenfläche aufweist, die sich von dem distalen Ende des Elementmaterials zu dem inneren Ende des Elementmaterials erstrecken, und einen soliden Kernabschnitt (124), der einen zentralen Abschnitt des distalen Endelements umfasst und sich entlang der Längsmittelachse von dem distalen Ende des Elements zu dem inneren Ende des Elements erstreckt, wobei der zentrale Abschnitt solide ist; und
Verdrehen wenigstens entweder des distalen Endes des Elementmaterials und/oder des inneren Endes des Elementmaterials um die Längsachse, sodass die Nuten schraubenförmig um die Längsachse gebildet werden, um ein verdrehtes distales Endelement zu bilden.

14. Verfahren nach Anspruch 13, wobei der Schritt des Verdrehens das distale Endelementmaterial so verformt, dass die Form des verdrehten distalen Endelements (118) beibehalten wird.

15. Verfahren nach Anspruch 13 oder 14, wobei der Schritt des Verdrehens das Drehen des distalen Endes des distalen Endelementmaterials (120) um wenigstens 90 Grad relativ zu dem inneren Ende des distalen Endelementmaterials (122) umfasst.

## Revendications

1. Article d'inhalation (110) comprenant :
un corps (112) s'étendant le long d'un axe central longitudinal (A) depuis une extrémité d'embout buccal (113) jusqu'à une extrémité distale (114) ;
une cavité de capsule (116) définie au sein du corps ; et
un élément d'extrémité distale (118) disposé au niveau de l'extrémité distale de l'article d'inhalation et s'étendant jusqu'à la cavité de capsule, l'élément d'extrémité distale comprenant une extrémité distale d'élément (120), une extrémité interne d'élément (122), une partie de noyau solide (124), et au moins deux rainures (126), dans lequel la partie de noyau solide comprend une partie centrale de l'élément d'extrémité distale, dans lequel la partie centrale est solide et s'étend le long de l'axe central longitudinal depuis l'extrémité distale d'élément jusqu'à l'extrémité interne d'élément, dans lequel les au moins deux rainures sont des rainures hélicoïdales qui tournent autour de la partie de noyau solide le long de l'axe central longitudinal depuis l'extrémité distale d'élément jusqu'à l'extrémité interne d'élément, et dans lequel les au moins deux rainures hélicoïdales s'étendent le long d'une surface externe (128) de l'élément d'extrémité distale.

2. Article d'inhalation (110) selon la revendication 1, dans lequel l'élément d'extrémité distale (118) est formé d'un matériau biodégradable.

3. Article d'inhalation (110) selon l'une quelconque des revendications précédentes, dans lequel l'élément d'extrémité distale (118) comprend un matériau fibreux.

4. Article d'inhalation (110) selon l'une quelconque des revendications précédentes, dans lequel l'élément d'extrémité distale (118) est formé d'un matériau poreux.

5. Article d'inhalation (110) selon l'une quelconque des revendications précédentes, dans lequel l'élément d'extrémité distale (118) est formé d'un matériau cellulosique.

6. Article d'inhalation (110) selon l'une quelconque des revendications précédentes, dans lequel l'élément d'extrémité distale (118) est formé d'un matériau d'acétate de cellulose.

7. Article d'inhalation (110) selon l'une quelconque des revendications 1 à 4, dans lequel l'élément d'extrémité distale (118) est formé d'un matériau d'acide polylactique.

8. Article d'inhalation (110) selon l'une quelconque des revendications précédentes, dans lequel les rainures (126) tournent d'au moins 90 degrés depuis l'extrémité distale d'élément (120) jusqu'à l'extrémité interne d'élément (122).

9. Article d'inhalation (110) selon l'une quelconque des revendications précédentes, dans lequel l'extrémité distale d'élément (120) est sensiblement alignée avec l'extrémité distale (114) .

10. Article d'inhalation (110) selon l'une quelconque des revendications précédentes, dans lequel une capsule (130) est disposée au sein de la cavité de capsule (116) de l'article d'inhalation.

11. Article d'inhalation (110) selon la revendication 10, dans lequel une capsule (130) contient des particules pharmaceutiquement actives comprenant de la nicotine, les particules pharmaceutiquement actives ayant un diamètre aérodynamique médian en masse d'environ 5 micromètres ou moins, ou dans une plage d'environ 0,5 micromètre à environ 4 micromètres, ou dans une plage d'environ 1 micromètre à environ 3 micromètres.

12. Système d'inhalation comprenant :
l'article d'inhalation (110) selon l'une quelconque des revendications précédentes ; et
un support (150) configuré pour recevoir l'article d'inhalation.

13. Procédé de fabrication d'un élément d'extrémité distale (118) pour un article d'inhalation (110), le procédé comprenant les étapes de :
fourniture d'un matériau d'élément d'extrémité distale ayant un axe longitudinal (A), une surface externe (142), une extrémité distale de matériau d'élément (120) et une extrémité interne de matériau d'élément (122), dans lequel le matériau d'élément d'extrémité distale comprend au moins deux rainures (126) sur la surface externe s'étendant depuis l'extrémité distale de matériau d'élément jusqu'à l'extrémité interne de matériau d'élément_et une partie de noyau solide (124) comprenant une partie centrale de l'élément d'extrémité distale et s'étendant le long de l'axe central longitudinal depuis l'extrémité distale de l'élément jusqu'à l'extrémité interne d'élément, dans lequel la partie centrale est solide ; et
torsion d'au moins l'une parmi l'extrémité distale de matériau d'élément et l'extrémité interne de matériau d'élément autour de l'axe longitudinal de sorte que les rainures sont formées de manière hélicoïdale autour de l'axe longitudinal pour former un élément d'extrémité distale torsadé.

14. Procédé selon la revendication 13, dans lequel l'étape de torsion déforme le matériau d'élément d'extrémité distale de sorte que la forme de l'élément d'extrémité distale torsadé (118) est maintenue.

15. Procédé selon la revendication 13 ou 14, dans lequel l'étape de torsion comprend la rotation de l'extrémité distale (120) du matériau de l'élément d'extrémité distale d'au moins 90 degrés par rapport à l'extrémité interne (122) du matériau de l'élément d'extrémité distale.
